(19) Europäisches Patentamt
European Patent Office
Office européen des brevets

(11) Publication number: **0 172 431 B1**

(12) **EUROPEAN PATENT SPECIFICATION**

(45) Date of publication of patent specification: 21.08.91

(51) Int. Cl.⁵: **C07C 29/15**, C07C 31/04, C07C 31/08, C07C 31/10, C07C 31/02

(21) Application number: 85109214.8

(22) Date of filing: 23.07.85

(54) Process for producing alcohols from synthesis gas.

(30) Priority: 30.07.84 US 636000

(43) Date of publication of application:
26.02.86 Bulletin 86/09

(45) Publication of the grant of the patent:
21.08.91 Bulletin 91/34

(84) Designated Contracting States:
BE DE FR GB IT NL

(56) References cited:
EP-A- 0 119 609
FR-A- 1 058 797

(73) Proprietor: THE DOW CHEMICAL COMPANY
2030 Dow Center Abbott Road P.O. Box 1967
Midland Michigan 48640-1967(US)

(72) Inventor: Stevens, Rex R.
4311 Andre
Midland, MI 48640(US)

(74) Representative: Casalonga, Axel et al
BUREAU D.A. CASALONGA - JOSSE Moras-
sistrasse 8
W-8000 München 5(DE)

## Description

This invention relates to a Fischer-Tropsch process for making alcohols and describes the catalyst composition and conditions of the process.

Almost as old as the Fischer-Tropsch process for making hydrocarbons is the Fischer-Tropsch process for making alcohols. The reaction is carried out by passing a mixture of carbon monoxide and hydrogen over a catalyst for the hydrogenation of the carbon monoxide. A typical review article is R. B. Anderson et' al., Industrial and Engineering Chemistry, Vol. 44, No. 10, pp. 2418-2424. This paper lists a number of catalysts containing zinc, copper, chromium, manganese, thorium, iron, occasionally promoted with alkali or other materials for making various alcohols. The authors state that ethyl alcohol is a major constituent, the yield of methanol is usually very small and a tentative summary of factors favoring the production of alcohols are high pressure, low temperature, high space velocity, high recycle ratio and carbon monoxide-rich synthesis gas.

Molybdenum is known to be catalytic for the Fischer-Tropsch process and is taught in U.S. Patents 4,151,190 and 4,199,522.

U.S. Patent 2,490,488 discloses that molybdenum sulfide methanation catalysts acquire Fischer-Tropsch activity when promoted with an alkaline compound of an alkali metal. The example shows a 30 percent selectivity to $C_3$ and higher hydrocarbons and oxygenates. Of this 30 percent, no more than 44 percent boils near or above 65°C, the boiling point of methanol. Accordingly the maximum possible alcohol selectivity is no more than 13.2 percent (44 percent of 30 percent).

U.S. Patent 2,539,414 describes a Fischer-Tropsch process with molybdenum carbide catalysts. It teaches that the catalyst may be used to form oxygenates and at column 3, lines 66-71 teaches that one might get alcohols or hydrocarbons by varying the conditions.

A number of references teach production of alcohols using rhodium catalysts which may contain molybdenum as an optional ingredient. U.S. Patent 4,014,913 discloses a catalyst containing rhodium and thorium, uranium and iron, molybdenum or tungsten for the production of ethanol. U.S. Patent 4,096,164 discloses the use of rhodium in combination with molybdenum or tungsten. Example A discloses that use of a molybdenum-on-silica catalyst yielded 4.4 percent oxygenates.

To make a commercially significant alcohol process, one must use a catalyst and conditions which are highly efficient. To be efficient the catalyst must yield a high ratio of mass of product per given mass of catalyst in a given period of time. The catalyst must be stable and active for long periods of time between regenerations. This may be particularly difficult to accomplish when the $H_2/CO$ ratio of the feed gas is low, such as less than 2 to 1. Ideally the catalyst will be highly selective to a commercial product (a) to avoid purification or removal and disposal of by-products and (b) to avoid separation into two or more product streams.

When the mixed alcohols product is to be used as a fuel replacement or a fuel additive, it may be desirable that the ratio of $C_1$ to $C_2$ and higher alcohols be no greater than a certain amount. The ratio of $C_1$ to $C_2$ and higher alcohols means the weight ratio of methanol to higher alcohols such as ethanol, propanols, butanols, and others, taken as a whole. This number may be calculated by determining the weight fraction of methanol in the mixed alcohols. When the weight fraction of methanol is x, the ratio of $C_1$ to $C_2$ and higher alcohols is

$$\frac{x}{1-x}.$$

Since and higher alcohols, in its broadest definition refers to alcohols which are not detected by conventional analytical techniques, a more meaningful approximation of the $C_1$ to $C_2$ and higher ratio of methanol to higher alcohols includes only the $C_2$-$C_5$ alcohols. Alcohols bound as esters or ethers are not included in either the $C_1$ or $C_2$ and higher numbers.

This invention concerns preparing alcohols from $H_2/CO$ synthesis gas. It is possible with this process to make a high yield of alcohols with a catalyst which is selective to alcohols boiling in the range of motor gasoline and which is stable, particularly at low $H_2/CO$ ratios, and which is active over long periods of time. The resulting mixed alcohol fraction has a lower $C_1$ to $C_2$ and higher alcohols ratio than obtainable with a straight molybdenum catalyst. The results are obtained without lowering the activity of the catalyst and without increasing the sulfur level in the product stream.

The present invention concerns a process for making alcohols boiling in the range of motor gasoline in at least 20 percent carbon dioxide free carbon selectivity wherein the weight ratio of $C_1$ to $C_{2-5}$ alcohol fraction is less that about 1.0 by reacting a mixture of:

(1) hydrogen;

(2) carbon monoxide; and

(3) a catalyst having (a) as a first component, molybdenum in free or combined form; (b) as a second component, a promoter of an alkali or alkaline earth element, in free or combined form; and (c) as a third component, at least one element of iron, cobalt or nickel, in free or combined form, wherein the atomic ratio of the first component to the third component is from about 1:4 to about 4:1 with the exception of a catalyst wherein the Mo/Co atomic ratio is 3:1.

EP-A- 0 119 609 which possesses an earlier priority date but is published only after the priority date of the present application, discloses a similar process without, however, giving any details about the atomic ratio of the first component to the third component. In an example a catalyst was used wherein the Mo/Co atomic ratio was 3:1.

Optionally, a fourth component, a support, may be present.

Contrary to the prior art, high yields and selectivity may be obtained without the use of rhodium, copper, ruthenium or zinc. An advantage of the process is that high production rates may be obtained at high selectivities. Under preferred conditions, these catalysts may yield high $C_1$-$C_5$ alcohol productivity. Up to about 1.4 weight units $C_1$-$C_5$ alcohol/hr/weight unit of catalyst may be achieved. With cobalt, iron or nickel added to the catalyst the ratio of $C_1$ to $C_2$-$C_5$ alcohols may be considerably lower than for the same catalyst without the iron, nickel or cobalt, while still retaining the high catalyst activity and low sulfur level mixed alcohol fraction. Because of the high selectivity, complex purification steps may be avoided and the alcohol product may have a low acid content and have a high octane blending value. This may permit blending into motor fuels without elaborate processing. In addition, contrary to what is experienced with a molybdenum catalyst, as one increases the temperature, the ratio of $C_1$ to $C_2$-$C_5$ alcohols may stay the same or may even decrease.

The hydrogen and carbon monoxide required for this process can be obtained by methods known in the art. Examples are gasification of hydrocarbonaceous materials such as coal, high specific gravity oils, or natural gas; as a by-product of partial combustion cracking of hydrocarbons; by steam reforming of liquid or gaseous hydrocarbons; through the water-gas shift reaction; or some combination of these. The two components may also be generated separately and combined for the subject reaction. The molar ratio of hydrogen to carbon monoxide in the feed gas which contacts the catalyst ranges generally from about 0.25 to about 100, preferably from about 0.5 to about 5 and most preferably from about 0.7 to about 3. A most preferred range of from about 0.7 to about 1.2 holds for unsupported $Co/MoS_3$ catalysts.

Generally, the selectivity to alcohols is dependent on the pressure. In the normal operating ranges, the higher the pressure at a given temperature, the more selective the process will be to alcohols. The minimum contemplated pressure is about 500 psig (3.55 MPa). The preferred minimum is about 750 psig (5.27 MPa) with about 1,000 psig (7.00 MPa) being a more preferred minimum. While about 1,500 psig (10.45 MPa) to about 4,000 psig (27.7 MPa) is the most desirable range, higher pressures may be used and are limited primarily by cost of the high pressure vessels and compressors needed to carry out the higher pressure reactions. A typical maximum is about 10,000 psig (69.1 MPa) with about 5,000 psig (34.6 MPa) a more preferred maximum. Thus, the broadest range is about 500 psig (3.55 MPa) to about 10,000 psig (69.1 Mpa).

The selectivity to alcohols is also a function of temperature and is interrelated with the pressure function. The minimum temperature used is governed by productivity considerations and the fact that at temperatures below about 200° C volatile catalytic metal carbonyls may form. Accordingly, the minimum temperature is generally around 200° C. For a given catalyst, at a constant pressure, as the temperature increases, the selectivity to alcohols decreases. A preferred maximum temperature is about 400° C. A more preferred maximum is about 350° C. Thus the preferred range is about 200 to about 400° C. However, the most preferred range of operation is from about 240° C to about 325° C.

The $H_2$/CO gas hourly space velocity (GHSV) is a measure of the volume of hydrogen plus carbon monoxide gas at standard temperature and pressure passing a given volume of catalyst in an hour's time. This may range from about 100 to about 20,000 and preferably from about 2,000 to about 5,000. Selectivity to the alcohols generally increases as the space velocity increases. However, conversion of carbon monoxide decreases as space velocity increases.

Preferably at least a portion of the unconverted hydrogen and carbon monoxide in the effluent gas from the reaction, more preferably after removal of product alcohols, water and carbon dioxide formed and even more preferably any hydrocarbons formed, may be recycled to the reaction. The amount of recycle is expressed as the recycle ratio which is the ratio of moles of gases in the recycle stream to the moles of gases in the fresh feed stream. A recycle ratio of zero is within the scope of the invention with at least some recycle preferred. A recycle ratio of at least about one is more preferred and at least about three is most preferred.

With preferred catalysts and under preferred conditions of temperatures, pressures, $H_2/CO$ ratio, GHSV and recycle ratio, about 0.1 weight units of alcohols or more per hour may be formed per weight unit of catalyst. Under the more preferred conditions of about 310° C, 1500 psig (10.45 MPa), a GHSV of 3800 and a $H_2/CO$ ratio of about 1:1, with a 2Mo/Co catalyst, about 0.3 weight units of alcohol or more per hour per weight unit of catalyst may be obtained. Under the most preferred conditions of about 340° C, 3000 psig (20.9 MPa), a GHSV of 13,000 and a $H_2/CO$ ratio of 1.1; with a 2Mo/Co catalyst about 1.4 weight units of alcohols or more per hour per weight unit of catalyst may be obtained.

Under preferred conditions, alcohols may be obtained in about an 85 percent carbon dioxide ($CO_2$) free carbon selectivity. The $CO_2$ free carbon selectivity is defined as 100 times the moles of carbon present in a product fraction divided by the total moles of carbon in all products which are not $CO_2$ or unconverted feed. For example, if one mole of ethanol is found in the alcohol fraction, this is counted as 2 moles of carbon. If 4 moles of carbon monoxide (CO) had been converted to products other than $CO_2$, the one mole of ethanol would result in ethanol being yielded at 50 carbon mole percent. Carbon dioxide and water are not counted as products in this calculation.

The first component of the catalyst is molybdenum in free or combined form.

The first component of the catalyst may be present in the catalyst in "free or combined form" which means that it may be present as a metal, an alloy or a compound of the element. Representative compounds include the sulfides, carbides, oxides, halides, nitrides, borides, salicylides, oxyhalides, carboxylates such as acetates, acetyl acetonates, oxalates, carbonyls, and the like. Representative compounds also include the elements in anionic form such as molybdates, phosphomolybdates and the like, and include the alkali, alkaline earth, rare earth and actinide series salts of these anions. The sulfides, carbonyls, carbides and oxides are preferred with the sulfides being most preferred.

The molybdenum may be present in an amount based on the weight of the total catalyst of at least about two percent, preferably at least about 5 percent with an upper limit of about 70 percent and preferably about 30 percent of the total catalyst.

The first and third components may be generally present as the sulfide. It is not necessary that any particular stoichiometric sulfide be present, only that the first and third components may be present in combination with sulfur. Some of the first or third component may be present in combination with other elements such as oxygen or as oxysulfides.

The third component of the catalyst preferably is at least one element of iron, cobalt or nickel, in free or combined form.

Because of the possiblity of the formation of nickel tetracarbonyl, cobalt and iron are preferred.

The third component of the catalyst may be present in the catalyst in "free or combined form" which means that it may be present as a metal, an alloy or a compound of the element. Representative compounds include the sulfides, carbides, oxides, halides, nitrides, borides, salicylides, oxyhalides, carboxylates such as acetates, acetylacetonates, oxalates, carbonyls, and the like. Representative compounds also include the elements combined with first component elements in anionic form such as iron, cobalt or nickel molybdates phosphomolybdates. The sulfides, carbonyls, carbides and oxides are preferred, with the sulfide being most preferred.

The iron, cobalt or nickel or mixtures thereof may be present in an amount based on the weight of the total catalyst of at least about two percent, preferably at least about 5 percent with an upper limit of about 70 percent and preferably about 30 percent of the total catalyst.

The first and third components may be present in the finished catalyst in an atomic ratio of about 1:10 to about 10:1. Preferably the first and third components are present in a ratio of from about 1:4 to about 4:1. With a coprecipitated Mo/Co sulfide catalyst an atomic ratio of Mo/Co of about 2:1 yields about a 1:5 weight ratio of methanol to $C_1$-$C_5$ alcohols and an atomic ratio of about 3:1 yields a weight ratio of about 1:3 methanol to $C_2$-$C_5$ alcohols.

The second component, a promoter, consists of one or more alkali elements or alkaline earth elements, in free or combined form. Alkali elements include: lithium, sodium, potassium, rubidium and cesium. Alkaline earth elements include: beryllium, magnesium, calcium, strontium and barium. Alkali elements and in particular, cesium and potassium, are preferred. Potassium is most preferred.

The promoter may be present in free or combined form as a metal, oxide, hydroxide, carbonate, sulfide or as a salt or a combination of these. The alkaline promoter is preferably present at a level sufficient to render the supported catalyst or the bulk catalyst more basic. The promoter is generally present in an amount of at least about 0.05 weight percent as a free element in the finished catalyst. Preferably it is present in an amount of at least about 0.5 percent and most preferably at least 2.0 percent. Large amounts up to about 30 percent of the promoter may be present. Preferably the promoter is present at less than 20 percent.

The promoter may be added as an ingredient to the other components or to the support, which is an optional fourth component, or may be part of one of the other components such as sodium or potassium molybdate or as an integral part of the support. For example, carbon supports prepared from coconut shells often contain small amounts of alkali metal oxides or hydroxides or the support may contain a substantial amount of the promoter, such as when the support is magnesia.

A fourth optional component of the catalyst is a support which may assume any physical form such as pellets, granules, beads, extrudates, and others. The support may be (a) coprecipitated with the active metal species, or (b) in powder form may be treated with the active metal species and then used or formed into the aforementioned shapes, or (c) may be formed into the aforementioned shapes and then treated with the active catalytic species.

The first three components may be dispersed on the support by methods known in the art. Examples include: impregnation from solution followed by conversion to the active species, vapor deposition, intimate physical mixing, sulfiding of other first or third component species, precipitation of sulfides in the presence of the support and the like. One or more of these methods may be used.

One alternative method of placing the first three components on the support is known as the incipient wetness technique. Water- or solvent-soluble salts of the metals to be dispersed on the support are chosen. The soluble salts which may be a single salt or more than one salt are dissolved in a quantity of solvent which may be aqueous, nonaqueous or a mixed solvent. A sufficient quantity of the resulting solution is added to the support in an amount no more than will be completely absorbed by the support. The solvent is then evaporated to leave the salt dispersed on the support. Depending on the solubility of the salt chosen and on the quantity of the element desired to be dispersed on the support, this process may be performed once or several times. Impregnations with two or more species may be performed by codissolving them in the solvent or by adding them separately in different quantities or types of solvent. If the species loaded on the support is not the desired one, the loaded support may be treated to convert it to the desired species. For example, oxides may be reduced, with reducing agents such as hydrogen; salts may be decomposed for example by heating, such as the decomposition of $(NH_4)_2MoS_4$ or $MoS_3$ to $MoS_2$; or one species may be converted to another by contact with a chemical agent, for example sulfiding. A catalyst may be sulfided by contact with a sulfur-containing agent such as $H_2S$.

Preferred methods of placing the first or third components on a support include, for example, impregnation with $(NH_4)_2MoS_4$ followed by decomposition with heat; precipitation of sulfides of the first and/or third components in contact with the support. Placing of the sulfided first and third components on a support is preferably followed by treatment with $H_2$ at elevated temperatures, usually with 20-50 ppm $H_2S$ present.

Exemplary support materials include: the aluminas, basic oxides, the silicas, carbons, or suitable solid compounds of magnesium, calcium, strontium, barium, scandium, yttrium, lanthanum and the rare earths, titanium, zirconium, hafnium, vanadium, niobium, tantalum, thorium, uranium, and zinc. Oxides are exemplary compounds. Preferably the supports are neutral or basic or may be rendered neutral or basic by addition of the alkaline promoters. The aluminas include the alpha, gamma, and eta types. The silicas include for example, silica gel, diatomaceous earth, and crystalline silicates.

The carbon supports, which are preferred supports, include activated carbons such as those prepared from coals and coal-like materials, petroleum-derived carbons and animal- and vegetable-derived carbons. Preferably the carbon support will have a surface area of 1-1500 m²/g, more preferably 10-1000 m²/g and most preferably 100-500 m²/g as measured by the BET nitrogen test. Preferably, micropores (<20 Å (<2 nm)) are minimized and at least twenty percent of the volume of the pores comprises pores having a diameter of from about 20 Å to about 600 Å (2-60 nm). Examples include coconut shell charcoal, coals, petroleum cokes, carbons formed by pyrolyzing materials such as vinylidene chloride polymer beads, coal, petroleum coke, lignite, bones, wood, lignin, nut shells, petroleum residues, charcoals, and others.

Based upon the weight of the total catalyst, the support when present generally comprises at least about 20 percent of the catalyst and generally not more than about 96 percent of the catalyst. Preferably the support comprises at least about 50 weight percent and most preferably at least about 70 weight percent of the catalyst.

The preferred form of the catalyst is the agglomerated sulfide. Certain forms of cobalt/molybdenum sulfide are more preferred. Most preferred is agglomerated, cobalt/molybdenum sulfide in which the cobalt and molybdenum sulfides are coprecipitated.

Methods for making sulfide catalysts are disclosed generally at pages 23-34 of Sulfide Catalysts Their Properties and Applications, O. Weisser and S. Landa, Pergamon Press, New York, 1973. Sulfide catalysts may be made by precipitating iron, cobalt or nickel sulfide in the presence of ammonium tetrathiomolybdate or other thiomolybdates, and thereafter thermally treating the mixture to convert the thiomolybdate salt to the sulfide; or as disclosed in U.S. Patents 4,243,553 and 4,243,554; or from purchased active combined first and third component sulfides.

Cobalt and molybdenum may be impregnated as salts on a support, then calcined to the oxide and then sulfided with $H_2S$ as taught in GB Patent publication 2,065,491. A cobalt/molybdenum sulfide may also be precipitated directly on to a support, but the unsupported cobalt/molybdenum sulfide is preferred. Other combinations of first and third component sulfides may be similarly made.

An unsupported catalyst preferably has a surface area of at least 10 $m^2/g$ and more preferably more than 20 $m^2/g$ as measured by the BET nitrogen surface area test.

A preferred method of making a cobalt/molybdenum sulfide, or other first and third component sulfide, consists of adding solutions of (1) ammonium tetrathiomolybdate or other equivalent salt and (2) a cobalt or nickel salt such as the acetate more or less simultaneously to (3) 30 percent acetic acid. This results in the coprecipitation of cobalt/molybdenum sulfide. By varying the ratios of cobalt and molybdenum or other salts in the solutions, one may vary the ratio of cobalt and molybenum or other elements in the sulfide catalyst. The cobalt/molybdenum sulfide or other sulfide may then be separated from the solvent, dried and blended with a second component promoter such as $K_2CO_3$ and agglomerating agents and/or pelleting lubricants, then pelleted and used as the catalyst in the process.

The alkali or alkaline earth promoter may be added to the active catalytic elements prior to, during or after the formation of the sulfide by physical mixing or solution impregnation. The active metal sulfide may then be combined with binders such as bentonite clay, and/or pelleting lubricants such as Sterotex® and formed into shapes for use as a catalyst.

Thus, the novel catalyst of the present process comprises:

(a) molybdenum in free or combined form;

(b) a promoter of an alkali or alkaline earth element, in free or combined form; and

(c) at least one element of iron, cobalt or nickel, in free or combined form, wherein the atomic ratio of the first component to the third component is from about 1:4 to about 4:1, with the exception of a catalyst wherein the Mo/Co atomic ratio is 3:1.

The finished catalyst may be used in a fixed bed, moving bed, fluid bed, ebullated bed or a graded bed wherein concentration and/or activity of the catalyst varies from inlet to outlet in similar manner to known catalysts. The catalyst may be used in powdered form or may be formed into shapes with or without a binder.

Catalysts used in the present process preferably contain less than 25 weight percent, based on the total weight of carbon oxide hydrogenation active metals, of other carbon oxide hydrogenation active metals and more preferably less than 20 weight percent and most preferably less than 2 weight percent. The catalyst may be essentially free of other carbon oxide hydrogenating components. By "essentially free" is meant that other carbon oxide hydrogenating components do not significantly alter the character or quantity of the alcohol fraction. For example, a significant change would be a five percent change in the amount of the alcohol fraction or a five percent change in the percentage of any alcohol in the alcohol fraction.

Carbon oxide hydrogenating components present in thus limited quantities or excluded are preferably those that contain chromium, manganese, copper, zinc, ruthenium and rhodium. More preferably, in addition to the above-mentioned components, those that contain: halogen, titanium, vanadium, cerium, thorium, uranium, iridium, palladium, platinum, silver and cadmium are excluded.

Under preferred conditions the catalyst is stable for long periods of time and under ideal conditions may be stable and active for as many as 6000 hours or more. Activity and selectivity are preferably substantially retained after 700 hours of operation, more preferably after 2000 hours and most preferably after 4000 hours operation. In the case of reduced oxide catalysts, declines in activity and selectivity may generally be regenerated by reduction with hydrogen after which the catalyst may regain most of its original activity and be used for another long period of time before regenerating again.

The catalysts are generally not adversely affected by up to 100 ppm sulfur in the $H_2/CO$ feed. However, no advantage is realized by the presence of sulfur, and generally sulfur must be removed from the mixed alcohols fraction. Accordingly, low sulfur levels in the feed are preferred.

At the conditions described above, the process yields substantial quantities of alcohols. Under preferred conditions, the weight units per hour of alcohols boiling in the range of motor gasoline per weight unit of catalyst may exceed 0.2. Under certain conditions, it may exceed 1.0 and may reach 1.4.

The alcohol fraction formed at greater than a 20 percent carbon dioxide free carbon selectivity boils in the motor gasoline range. The minimum boiling pure alcohol is methanol at 64.7° C. ASTM D-439 calls for a 225° C endpoint for automotive gasoline. Accordingly the alcohol fraction formed at greater than a 20 percent carbon dioxide free carbon selectivity may boil in the range of from about 60° C to about 225° C when distilled by ASTM D-86. It is not necessary that the entire liquid product boil in this range, but it is preferred. It is not necessary that the alcohol fraction meet all the distillation specifications for motor gasolines - only that it boil within the broad range of motor gasolines. For example, it need not be within 50 percent evaporated limits as set by ASTM D-439. Only 20 carbon mole percent of the total carbon dioxide free product must be alcohols that boil in this range. The alcohol fraction formed may be used as a motor fuel blending stock. Preferably, the alcohol fraction formed will have a research octane blending value in motor gasoline of greater than about 100, more preferaby greater than about 110 and most preferably greater than about 120.

Preferably, a $C_1$-$C_8$ alcohol fraction is formed in at least about 20 percent carbon dioxide free carbon selectivity and most preferably a $C_1$-$C_5$ alcohol fraction is formed in at least about 20 percent carbon dioxide free carbon selectivity.

The $C_1$-$C_5$ alcohol fraction may contain methanol, ethanol, 1-propanol, 1-butanol, 2-methyl-1-propanol, 1-pentanol, 2-methyl-1-butanol, but does not generally contain substantial tertiary alcohols. In addition to these named alcohols, the $C_1$-$C_8$ alcohol fraction may contain the $C_6$-$C_8$ alcohols wherein the hydroxyl group may be attached to a branched-chain carbon atom.

The present process for making mixed alcohols may yield a lower ratio of $C_1$ to $C_2$-$C_5$ alcohols in the alcohol fraction with a catalyst containing the first, second and third components and optionally the fourth component listed above, than with a catalyst containing the same first, second and optional fourth component but not having the third component. With just the first, second and optional fourth component, a typical $C_1$ to $C_2$-$C_5$ weight ratio may be 1.1 or more. With the present catalyst the $C_1$ to $C_2$-$C_5$ weight ratio may be less than one, preferably less than about 0.8, more preferably less than about 0.5, and most preferably less than about 0.4 and possibly even about 0.25 or lower.

Primarily the $C_2$-$C_5$ alcohol that increases is ethanol. The weight percentage of ethanol made without the third component in the catalyst is typically less than 25 percent of the total $C_1$-$C_5$ alcohols. In the presence of an iron-, cobalt- or nickel-containing catalyst of the same character otherwise, the ethanol may be greater than 25 weight percent, preferably greater than 30 weight percent and most preferably greater than 40 weight percent of the $C_1$-$C_5$ alcohol fraction.

The present catalysts preferably contain the first component and third component in an atomic ratio of less than about 5:1. Preferably, the atomic ratio is less than 3:1 and most preferably about 2:1, 1:1 or less.

Coprecipitated cobalt/molybdenum sulfide is the preferred combination of the first and third components. The sulfur content may or may not be stoichiometric as there are many sulfides of these two metals.

Under preferred conditions, the amount of water formed is substantially less than the amount of alcohols formed. Typically there is less than 20 weight percent and preferably less than 10 weight percent water based on the quantity of alcohol. This water may be removed by known techniques if the alcohol fraction is to be used as a motor fuel additive. If the water content is about two weight percent or less based on alcohols, the water may advantageously be removed by absorption on molecular sieves. At higher water contents one may use a water gas shift drying step as disclosed in British Patent Publications 2,076,015 and 2,076,423. A water gas shift catalyst tolerant to sulfur, and alcohol catalyst carry over should be used in the drying step. Halder Topsoe SSK is exemplary.

The product mixture, as formed under preferred conditions, contains only small portions of other oxygenated compounds besides alcohols. These other compounds are not deleterious to using the product in motor fuels.

In all cases, the alcohol fraction is formed in at least about 20 percent carbon dioxide free carbon selectivity. Preferably the alcohol fraction is formed in at least about 30 percent carbon dioxide free carbon selectivity, more preferably greater than about 50 percent and ideally can be greater than about 70 percent.

Preferably the co-products formed with the alcohol fraction are primarily gaseous products. That is $C_1$-$C_4$ hydrocarbons. By hydrocarbons, it is meant that heteroatoms such as oxygen, sulfur and nitrogen are not present in the molecule. Preferably $C_5$ and higher hydrocarbons are coproduced at less than about 20 percent carbon dioxide free carbon selectivity, more preferably at less than 10 percent and most preferably at less than 5 percent. Lower amounts of normally liquid hydrocarbons make the normally liquid alcohols easier to separate from by-products.

Generally, alcohol selectivity may be increased by increasing pressure, space velocity, product gas recycle ratio and by decreasing $H_2/CO$ feed ratio and temperature.

The following examples are provided to illustrate the present process.

CATALYSTS

Comparison A

7

A solution of 180 g of $(NH_4)_6 Mo_7O_{24} \cdot 4H_2O$ in 500 cm$^3$ of water containing 100 cm$^3$ of concentrated $NH_4OH$ reacts with a small excess of $(NH_4)_2S$ (about 1300 cm$^3$ of 22 percent $(NH_4)_2S$ in water). The reaction mixture is stirred at 60°C for one hour and evaporated to dryness at 60-70°C. A portion of the resulting $(NH_4)_2MoS_4$ is calcined at 500°C for one hour in an inert atmosphere such as nitrogen to form $MoS_2$. The resulting $MoS_2$ powder (6.6 g) is mixed with 2.0 g of bentonite clay, 1.0 g of $K_2CO_3$ and 0.4 g of a pelleting lubricant (Sterotex®) by grinding in a mortar and pestle. The product is used to make alcohols in the unpelleted powder state. No pretreatment of the catalyst is performed.

Example 1 (not an example of the invention)

A 10.0-g portion of the $MoS_2$ from Comparison A is mixed in a mortar and pestle with 8.4 g Co-$(CH_3CO_2)_2$ $4H_2O$ (cobalt acetate) and water sufficient to yield a thick paste. The paste is dried at 60°C and calcined at 500°C for one hour in an inert gas such as nitrogen to give a black powder with a Mo/Co atomic ratio of about 3:1.

Similar to Comparison A, 6.6 g of this powder are mixed with 2.0 g bentonite clay, 1.0 g of $K_2CO_3$ and 0.4 g of Sterotex® in a mortar and pestle. This catalyst is used in unpelleted powder form and is not pretreated.

Example 2

A coprecipitated cobalt/molybdenum sulfide is prepared with a Mo/Co atomic ratio of about 2:1. Fifteen grams of $(NH_4)_6 Mo_7O_{24} \cdot 4H_2O$ (0.085 moles Mo) is dissolved in 106 cm$^3$ of 22 percent $(NH_4)_2S$ in water and stirred at 60°C for one hour to form $(NH_4)_2MoS_4$. A solution of 10.5 g of $Co(CH_3CO_2)_2$ (0.042 mole Co) in 200 cm$^3$ of water is prepared.

The two solutions are added simultaneously, dropwise to a stirred solution of 30 percent aqueous acetic acid in a baffled flask at 50°C over a one-hour period. After stirring for one additional hour the reaction mix is filtered and the filter cake dried at room temperature and then calcined for one hour at 500°C in an inert atmosphere such as nitrogen. Similar to Example 1, 6.6 g of the calcined cobalt/molybdenum sulfide is ground together with 2.0 g of bentonite clay, 1.0 of g $K_2CO_3$ and 0.4 g of Sterotex® lubricant in a mortar and pestle. This catalyst is used in unpelleted, powder form without pretreatment.

Example 3

This Example discloses the making of a coprecipitated cobalt/molybdenum sulfide with a Mo/Co atomic ratio of about 3:1.

The coprecipitated cobalt/molybdenum sulfide is prepared using the same procedure as Example 2 except that 7.1 g of $Co(CH_3CO_2)_2 \cdot 4H_2O$ (0.28 moles Co) is used. This catalyst is used in unpelleted, powder form without pretreatment.

Example 4

This Example discloses the use of a commercially available alkalized molybdenum/cobalt catalyst, Haldor Topsoe SSK, and available from Haldor Topsoe A/S of Denmark.

Example 5

This Example discloses the use of an alkalized Mo/Ni sulfide having a Mo/Ni atomic ratio of about 2:1.

Seventy-five grams (0.425 mole) of $(NH)_6 Mo, O_{24} \cdot H_2O$ is dissolved in 530 cm$^3$ of 22 percent aqueous $(NH_4)_2S$ at 60-70°C with stirring for one hour to give a solution of $(NH_4)_2MoS_4$. A second solution containing 53 g of nickel acetate (0.212 mole Ni) in 500 cm$^3$ of water is prepared. These two solutions are added dropwise over a 40-minute period to one liter of vigorously stirred 30 percent acetic acid. After stirring for one additional hour at 60°C, the resulting slurry is filtered. The black filter cake is washed with water and dried overnight at 100°C under nitrogen. The dry filter cake is calcined under nitrogen at 500°C for one hour. Similarly to Example 1, 6.6 g of the calcined Mo/Ni sulfide is ground with mortar and pestle with 2 g of bentonite clay, 1 g of $K_2CO_3$ and 0.4 g of Sterotex® pelleting lubricant. The catalyst is used in unpelleted powder form without pretreatment. Results are shown in the Table.

Example 6

This Example discloses the use of a Mo/Fe sulfide made by coprecipitation.

A barium acetate solution, prepared by dissolving 12.2 g (0.071 mole) of $Ba(OH)_2$ in 100 $cm^3$ of water containing 10 $cm^3$ of glacial acetic acid, is mixed with 100 $cm^3$ of aqueous solution containing 19.7 g (0.071 mole) of $FeSO_4$. The resulting precipitate was filtered off under nitrogen and discarded, leaving a solution of ferrous acetate. A solution of $(NH_4)_2MoS_4$ (0.142 mole) is prepared by dissolving 25 g of $(NH_4)_6Mo_7O_{24} \cdot 4H_2O$ in 180 $cm^3$ of 22 percent aqueous $(NH_4)_2S$ and stirring at 60°C for one hour. The solutions of ferrous acetate and ammonium tetrathiomolybdate are added simultaneously over a 30-minute period to a vigorously stirred solution of 75 $cm^3$ of glacial acetic acid in 225 $cm^3$ of water at 60°C. The resulting black slurry is stirred at 60°C for one hour and filtered. The black filter cake is washed, dried at 110°C overnight under nitrogen, and calcined at 500°C under nitrogen for one hour. The calcined Mo/Fe sulfide is blended in a mortar and pestle with bentonite clay, $K_2CO_3$ and Sterotex® to give a formulation containing 66 percent Mo/Fe sulfide, 20 percent clay, 10 percent $K_2CO_3$ and 4 percent Sterotex®. This catalyst (5 $cm^3$) is combined with 5 $cm^3$ of tabular alumina and loaded into the reactor.

Example 7

A coprecipitated cobalt molybdenum sulfide is made using the same procedure as Example 2 but using 0.085 moles of cobalt acetate. This gives a 1:1 atomic ratio of Mo:Co.

Preparation of Alcohols

In the general method of these Examples, the reactor consists of a one-half inch (1.27 cm) stainless steel tube packed with catalyst. The total volume of catalyst is about 6 $cm^3$. Premixed hydrogen, carbon monoxide, and nitrogen feed gases from cylinders are compressed and regulated at the pressures stated in the table. The feed gas mixture contains hydrogen and carbon monoxide at the stated molar ratios and about five percent by volume of nitrogen serving as an internal standard. About 50 ppm of $H_2S$ is also present in the feed gas.

The mixed feed gas passes through the bed of activated carbon at room temperature to remove iron and other carbonyl contaminants. The feed gas then passes at the stated hourly space velocities through the fixed bed reactor which is maintained at the stated reaction temperatures by an electric air recirculated oven and which is held at 1500 psig (10.45 MPa). The reactor effluent passes through a gas liquid separator at ambient temperature and the reaction pressure stated in series with a dry ice trap at ambient pressure. Both gas and liquid phases are analyzed to give the results in Table I.

TABLE I

| Example | A | 1[*] | 2 | 3 |
|---|---|---|---|---|
| Temp. (°C) | 265 | 295 | 305 | 295 |
| $H_2$/CO (molar ratio) | 1.04 | 0.98 | 0.98 | 0.98 |
| GHSV | 1200 | 2200 | 1300 | 1050 |
| CO Conversion (%) | 33.1 | 10.3 | 39.0 | 29.2 |
| Wt. Units CO converted per wt. unit of catalyst per hr | 0.19 | 0.12 | 0.23 | 0.13 |
| $CO_2$ produced[1] (%) | 31.3 | 18.6 | 33.5 | 31.3 |
| Selectivities[2] (%) Gas Phase | | | | |
| $CH_4$ | 20.2 | 7.0 | 12.6 | 11.3 |
| $C_2$ and higher hydrocarbons | 4.3 | 3.2 | 5.7 | 3.2 |
| Subtotal | 24.5 | 10.2 | 18.2 | 14.5 |
| Liquid Phase | | | | |
| Methanol | 32.3 | 37.8 | 16.1 | 22.7 |
| Ethanol | 31.8 | 29.5 | 39.9 | 40.7 |
| Propanols | 7.7 | 7.8 | 14.9 | 12.7 |
| Butanols | 1.6 | 5.3 | 4.3 | 3.5 |
| Pentanols | 0.2 | 2.4 | 0.5 | 1.2 |
| Subtotal | 73.6 | 82.8 | 75.7 | 80.8 |
| Weight Ratio $C_1/C_2-C_5$ alcohols | 1.13 | 1.24 | 0.39 | 0.57 |
| Other oxygenates[3] and hydrocarbons | 1.9 | 7.0 | 6.0 | 4.7 |
| $H_2O$[4] (wt. %) | 2.7 | 1.4 | 1.8 | 2.3 |

* Not an example of the invention

EP 0 172 431 B1

TABLE I (cont'd)

| Example | 4 | 5 | 6 | 7 |
|---|---|---|---|---|
| Temp. (°C) | 350 | 300 | 321 | 300 |
| $H_2/CO$ (molar ratio) | 1.02 | 1.03 | 1.04 | 1.04 |
| GHSV | 614 | 1330 | 1480 | 1259 |
| CO Conversion (%) | 12.7 | 33.1 | 27.1 | 34.7 |
| Wt. units CO converted per wt units of cata- lyst per hr | 0.04 | 0.26 | 0.27 | 0.20 |
| $CO_2$ produced[1] (%) | 40.1 | 32.9 | 36.9 | 33.6 |
| Selectivities[2] (%) Gas Phase | | | | |
| $CH_4$ | 21.7 | 18.0 | 7.4 | 12.3 |
| $C_2$ and higher hydrocarbons | 4.9 | 2.7 | 16.7 | 3.0 |
| Subtotal | 26.6 | 20.7 | 24.1 | 15.3 |
| Liquid Phase | | | | |
| Methanol | 17.7 | 15.2 | 6.9 | 16.4 |
| Ethanol | 15.2 | 41.8 | 21.0 | 41.3 |
| Propanols | 16.7 | 11.5 | 17.9 | 14.3 |
| Butanols | 10.9 | 1.4 | 10.8 | 4.1 |
| Pentanols | 5.0 | 1.5 | 7.1 | 0.7 |
| Subtotal | 65.5 | 71.4 | 63.7 | 76.8 |
| Weight Ratio $C_1/C_2$-$C_5$ alcohols | 0.60 | 0.39 | 0.19 | 0.34 |
| Other oxygenates[3] and hydrocarbons | 7.9 | 7.9 | 12.2 | 7.9 |
| $H_2O$[4] (wt. %) | 4.6 | 1.9 | 6.7 | 1.7 |

[1]100 x moles of $CO_2$ formed for each mole of CO converted in the reactor.

[2]Selectivities, except for $CO_2$, are based on carbon mole selectivity on a $CO_2$ free basis.

[3]Assumed a carbon number of 4 for other oxygenates.

[4]Water is calculated as weight percent of the liquid phase.

## Claims

1. A process for making alcohols boiling in the range of motor gasoline in at least 20 percent carbon dioxide free carbon selectivity wherein the weight ratio of $C_1$ to $C_{2-5}$ alcohol fraction is less than about 1.0 by reacting a mixture of:
   (1) hydrogen,

11

(2) carbon monoxide; and

(3) a catalyst having (a) as a first component, molybdenum in free or combined form, (b) as a second component, a promoter of an alkali or alkaline earth element in free or combined form; and (c) as a third component, at least one element of iron, cobalt or nickel, in free or combined form wherein the atomic ratio of the first component to the third component is from about 1:4 to about 4:1 with the exception of a catalyst wherein the Mo/Co atomic ratio is 3:1.

2. The process of Claim 1 wherein the catalyst has as a fourth component a support.

3. The process of claim 1 or 2 wherein the third component of the catalyst is iron.

4. The process of Claim 3 wherein the atomic ratio of the first component to the third component is from about 1:1 to about 3:1.

5. The process according to claims 1-4 wherein the first and third components are molybdenum and cobalt respectively, in free or combined form.

6. The process according to claims 1-5 wherein the first and third components are present as coprecipitated sulfides.

7. The process according to claims 1-6 wherein the mixture of hydrogen and carbon monoxide contains a molar ratio of $H_2/CO$ of less than 2:1.

8. A catalyst comprising:
(a) molybdenum in free or combined form;
(b) a promoter of an alkali or alkaline earth element, in free or combined form; and
(c) at least one element of iron, cobalt or nickel, in free or combined form, wherein the atomic ratio of the first component to the third component is from about 1:4 to about 4:1, with the exception of a catalyst wherein the Mo/Co atomic ratio is 3:1.

**Revendications**

1. Procédé de fabrication d'alcools bouillant dans le domaine des points d'ébullition de l'essence pour moteur, avec une sélectivité rapportée au carbone, dioxyde de carbone mis à part, valant au moins 20%, et dans lequel le rapport pondéral du méthanol à la fraction d'alcools en $C_{2-5}$ est inférieur à environ 1,0, par réaction d'un mélange de :
(1) hydrogène,
(2) monoxyde de carbone, et
(3) un catalyseur comportant, (a) comme premier composant, du molybdène sous forme libre ou combinée, (b) comme second composant, un promoteur constitué d'un élément alcalin ou alcalino-terreux, sous forme libre ou combinée, et (c) comme troisième composant, au moins un élément choisi parmi fer, cobalt et nickel, sous forme libre ou combinée, et dans lequel le rapport atomique du premier composant au troisième composant vaut d'environ 1:4 à environ 4:1, à l'exception d'un catalyseur dans lequel le rapport atomique Mo/Co vaudrait 3:1.

2. Procédé selon la revendication 1, dans lequel le catalyseur comporte un support comme quatrième composant.

3. Procédé selon la revendication 1 ou 2, dans lequel le troisième composant du catalyseur est du fer.

4. Procédé selon la revendication 3, dans lequel le rapport atomique du premier composant au troisième composant vaut d'environ 1:1 à environ 3:1.

5. Procédé selon les revendications 1 à 4, dans lequel les premier et troisième composants sont respectivement du molybdène et du cobalt, sous forme libre ou combinée.

6. Procédé selon les revendications 1 à 5, dans lequel les premier et troisième composants se trouvent sous forme de sulfures co-précipités.

7. Procédé selon les revendications 1 à 6, dans lequel l'hydrogène et le monoxyde de carbone contenus dans le mélange s'y trouvent en un rapport molaire $H_2/Co$ inférieur à 2:1.

8. Catalyseur comprenant :
   (a) du molybdène sous forme libre ou combinée,
   (b) un promoteur constitué d'un élément alcalin ou alcalino-terreux, sous forme libre ou combinée, et
   (c) au moins un élément choisi parmi le fer, le cobalt et le nickel, sous forme libre ou combinée,
   et dans lequel le rapport atomique du premier composant au troisième composant vaut d'environ 1:4 à environ 4:1. à l'exception d'un catalyseur dans lequel le rapport atomique Mo/Co vaudrait 3:1.

## Patentansprüche

1. Verfahren zur Herstellung von Alkoholen, die im Bereich von Motorbenzin sieden, in mindestens 20 % Kohlendioxidfreier Kohlenstoff-Selektivität, worin das Gewichtsverhältnis von $C_1$ zu $C_{2-5}$ Alkoholfraktion kleiner als ungefähr 1,0 ist, durch zur Reaktionbringen einer Mischung von
   (1) Wasserstoff,
   (2) Kohlenmonoxid; und
   (3) einem Katalysator mit (a) als einer ersten Komponente Molybdän in freier oder kombinierter Form, (b) als einer zweiten Komponente einen Promotor eines Alkali- oder Erdalkalielements in freier oder kombinierter Form und (c) als einer dritten Komponente mindestens einem Element aus Eisen, Kobalt oder Nickel in freier oder kombinierter Form, worin das Atomverhältnis der ersten Komponente zur dritten Komponente ungefähr 1:4 bis ungefähr 4:1 beträgt, mit der Ausnahme eines Katalysators, bei dem das Mo/Co-Atomverhältnis 3:1 ist.

2. Verfahren nach Anspruch 1, worin der Katalysator als eine vierte Komponente ein Trägermaterial enthält.

3. Verfahren nach Anspruch 1 oder 2, worin die dritte Komponente des Katalysators Eisen ist.

4. Verfahren nach Anspruch 3, worin das Atomverhältnis der ersten Komponente zur dritten Komponente ungefähr 1:1 bis ungefähr 3:1 beträgt.

5. Verfahren nach einem der Ansprüche 1 bis 4, worin die ersten und dritten Komponenten Molybdän und Kobalt in freier oder kombinierter Form sind.

6. Verfahren nach einem der Ansprüche 1 bis 5, worin die ersten und dritten Komponenten als copräzipitierte Sulfide anwesend sind.

7. Verfahren nach einem der Ansprüche 1 bis 6, worin die Mischung von Wasserstoff und Kohlenmonoxid ein molares Verhältnis von $H_2/CO$ von weniger als 2:1 enthält.

8. Katalysator umfassend
   (a) Molybdän in freier oder kombinierter Form;
   (b) einen Promotor eines Alkali- oder Erdalkalielements in freier oder kombinierter Form; und
   (c) mindestens ein Element aus Eisen, Kobalt oder Nickel in freier oder kombinierter Form,
   worin das Atomverhältnis der ersten Komponente zur dritten Komponente ungefähr 1:4 bis ungefähr 4:1 beträgt, mit Ausnahme eines Katalysators, worin das Mo/Co-Atomverhältnis 3:1 ist.